# EUROPEAN PATENT APPLICATION

(11) **EP 0 696 440 A1**
(43) Date of publication of application: **14.02.1996**
(21) Application number: 94202292.2
(22) Date of filing: 10.08.1994
(51) Int. Cl.: A61B 17/68, A61C 8/00

(54) **Chain of stably assemblable modular elements for bone or dental surgery**

(71) Applicant: OFFICINE BIOMECCANICHE S.r.l., I-20048 Carate Brianza, Milano (IT)
(72) Inventor: Foresti, Giancarlo, I-06066 Piegaro (Perugia) (IT)
(74) Representative: Martegani, Franco

(57) **Abstract**

A set of modular elements for bone surgery comprises, in combination: a plurality of intermediate elements (11) arranged between a nose element (12) and a head element (13). Each of said intermediate (11) and head (13) elements comprises means for stable mutual intercoupling (14), (15), with the coupling means (15) of each modular element being suitable for being coupled with the coupling means (14) of another, adjacent modular element, said elements (11), (12), (13) having a tapered configuration with side hollows (20), (22) and drive means (19).

## Description

The present finding relates to a set of modular elements with can be stably intercoupled to each other in order to realize a remedy, a prosthesis, or a surgical tool, all for use in surgery in general and, in particular, in skeleton surgery.

In the field of bone surgery, which can be either of skeleton or of dental type, the use is known of special elements to be mutually intercoupled, e.g., by means of screws, in order to realize prostheses which can be of either provisional or final type.

Said elements get engaged inside a purposely provided seat (hollow) for them inside bone tissue, getting anchored to the walls of said seat thanks to the profile of the hollow being suitably adapted.

If the implantation surgery is correctly carried out, i.e., with no traumata for living matter, the interested bone heals embedding the applied element, which becomes an integral part of it (see "osteointegration").

Hence, such elements realize a prosthesis, or become an anchoring point, capable of receiving a support for a prosthesis.

As each procedure requires one or more elements with optimal size, the present technology offers a large number of expensive models with different length, diameter and shape.

Therefore, the surgeon must select and purchase the model featuring the optimal characteristics as a function of the expected surgery situation, however without neglecting that during the operation the need can arise for alternative solutions having to be adopted.

The wrong selection of the element model can in fact endanger the good outcome of the surgical procedure: too short elements will yield an insufficient anchoring, and too long elements will require laborious matching operations which could endanger the good outcome of the surgery.

In order to obviate the above said drawbacks, a surgical screw was proposed in the past, which is suitable for being used for a wide range of procedures, however without requiring complex fitting operations.

Said surgical screw is disclosed in the application for utility model patent No. MI91U 000361 filed in Italy on April 24th, 1991, to the name of Tech. Medical S.r.L. Instruments for Medical Supply.

Shortly, said screw for bone surgery, according to said utility model patent application, comprises a male portion and a female portion.

The male portion can be screwed down in the bone, and the residual female portion can be constrained to the male portion of a further, identical screw which will thereby become the extension of the preceding portion.

Thus, a composite screw can be obtained which has any desired length and obviates the drawback of the surgeon to provide in advance, before each surgical procedure, a large number of screws having different length.

Although it is highly innovative from the conceptual view point, in the step of practical implementation said screw proved to require some improvements as regards the following aspects.

First of all, it is regarded as being highly desirable that such a screw is so shaped as to facilitate, on the one hand, its penetration into the hole prepared on the bone, and, on the other hand, its stable and reliable anchoring inside the interior of said hole.

Additionally to the above, the shape of the head end screw in the system of mutually connected screws should be such as to facilitate, as far as possible, the application of a solid film-like layer of a suitable biocompatible substance capable of promoting the healing of the interfaced biological structure.

Finally, it would be highly desirable that, even in the presence of a large hollow in the bone, the system of mutually connected screws can be implanted immediately, i.e., without having to wait for the bone hollow to be reconstituted.

The general purpose of the present finding is of meeting the above listed requirements in a simple, cheap and functional way.

Such a purpose is achieved by a set of modular screws realized according to the appended claims.

The structural and functional characteristics of the finding and its advantages over the prior art will be still more evident from an examination of the following disclosure made by referring to the accompanying drawings which display a set of modular elements featuring the innovative principles of the same finding.

In the drawings:
Figure 1 shows a longitudinal sectional view illustrating the set of modular elements according to the finding;
Figure 2 shows an enlarged sectional view illustrating the nose of the set of elements of Figure 1;
Figure 3 shows a plan view according to arrow F of Figure 2;
Figure 4 shows an enlarged sectional view illustrating the nose of the set of elements of Figure 1;
Figure 5 shows a plan view according to arrow F1 of Figure 4;
Figure 6 shows an enlarged sectional view illustrating the shape of the intermediate elements of the set of elements of Figure 1;
Figure 7 shows a plan view according to arrow F3 of Figure 6;
Figures 8 and 9 show two similar views to the view of Figure 1, but illustrating the set of elements provided with one stop washer or two stop washers, respectively, to anchor the same set to the wall of a bone hollow;
Figure 10 shows a plan view illustrating a possible shape for a washer for the set of elements of Figures 8 and 9;
Figure 11 shows a longitudinal sectional view illustrating the set of modular elements according to the present finding, in which the mutual connection of two adjacent elements is realized by quick spring-like joint, rather than by screw-threading;
Figure 12 shows a longitudinal sectional view illustrating the shape of the elements of the set of elements of Figure 11; and
Figure 13 shows a plan view according to arrow F of Figure 12.

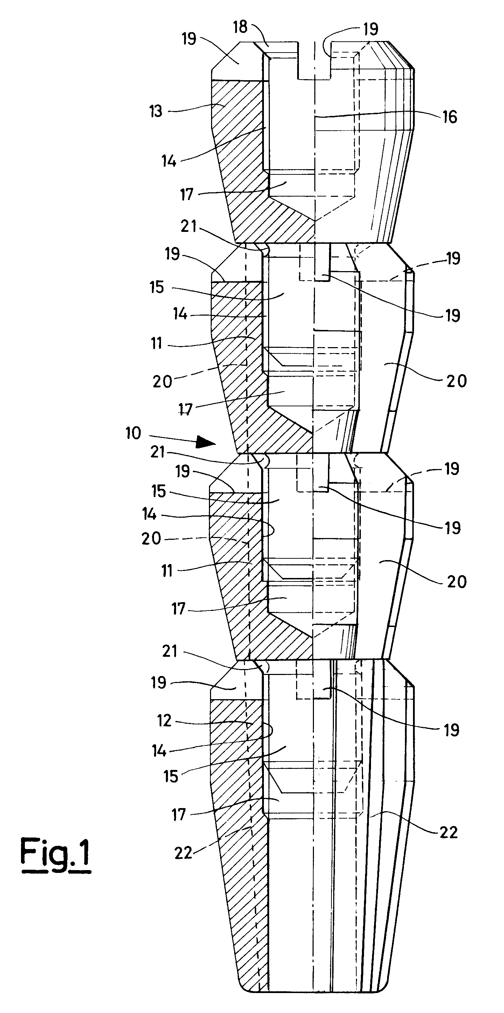

Referring first to Figure 1 of the accompanying drawings, the set of modular elements according to the instant finding is generally indicated with (10) and is structurally formed by a plurality of intermediate elements (11) arranged between a nose element (12) and a head element (13).

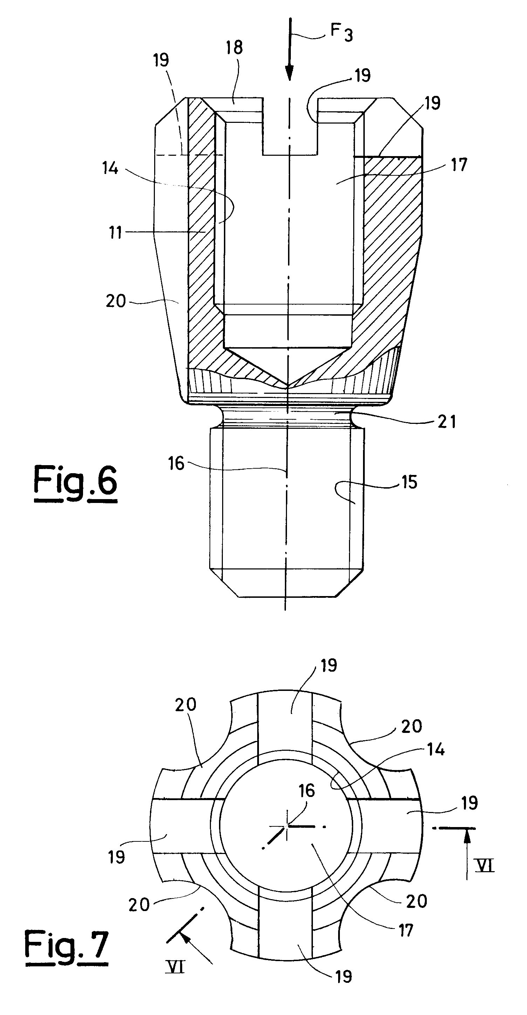

The intermediate elements (11) (Figures 6 and 7) comprise a screw-threaded female portion (14) and a screw-threaded male portion (15), arranged according to a common axis (16).

The female portion (14) comprises an axial blind hollow (17) with a countersunk (18) at the mouth.

Said female portion (14) is furthermore provided with the means for element drive which, in the exemplified case, are constituted by four groves (19) arranged in cross-like configuration (Figure 7).

Characteristically, the female portion (14) displays a cone frustum-shaped cross-section, with longitudinal hollows (20), which facilitates both the penetration and the anchoring of the element inside the hole previously provided in the bone tissue. The female portion (14) is linked to the male portion (15) at a ring-like groove (21) the purpose of which is explained hereinunder.

The screw-threadings of said female (14) and male (15) portions are identical to each other, so as to make it possible a plurality of intermediate elements (11) to be connected (as shown in Figure 1) in order that a composite structure (remedy, prosthesis, or surgical tool) having the necessary length can be attained.

Still for the same purpose, although the element (11) displays corresponding screw-threadings, the female portion (14) and the male portion (15) in the element (11) can be provided with respective lengths which can be the same, or the female portion (14) can be made longer than the male portion (15), or vice-versa.

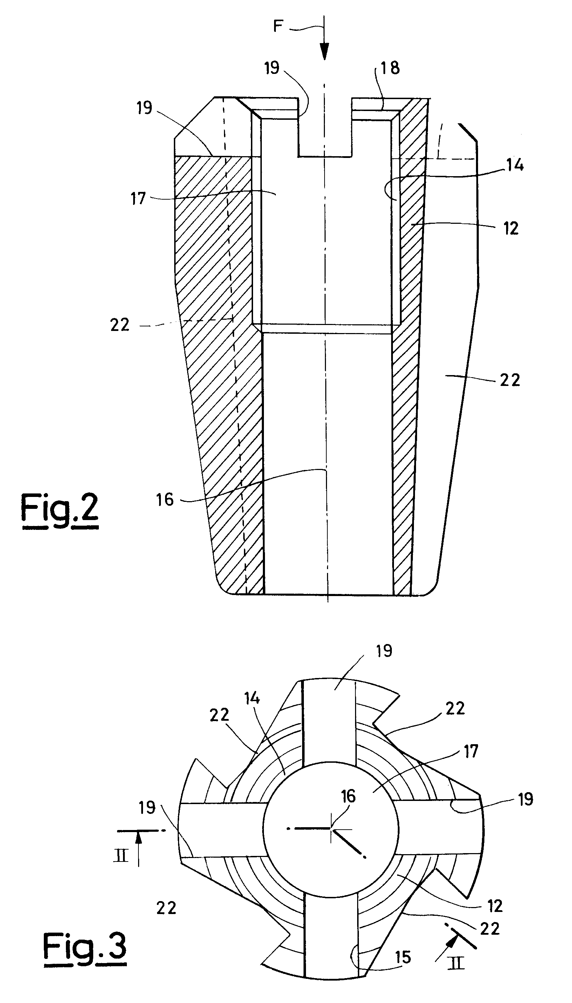

The nose element (12) of the set of modular elements is illustrated in greater detail in Figures 2, 3 of the drawings.

Characteristically, said nose element (12) has a cone-frustum configuration with longitudinal hollows (22) generally having a propeller-like shape, so as to favour the penetration and anchoring of the modular elements set inside the interior of the bone hollow.

As one will clearly see from Figure 2 of the accompanying drawings, the nose 12 is provided with a screw-threaded female portion (14) at all corresponding to the analogous portion of the intermediate elements (11) disclosed hereinabove.

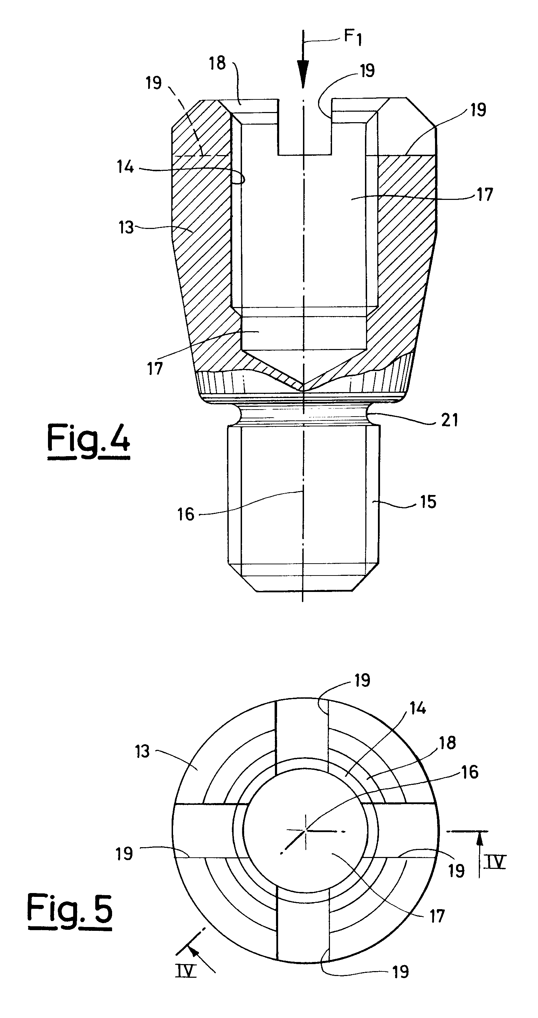

Figures 4 and 5 of the drawings display in greater detail the head element (13) of the set of modular elements, which, as compared to the intermediate elements (11), only differs from them in that it lacks the longitudinal hollows (20).

In fact, the side skirt of the head element (13) lacks any hollows, and can be submitted to the necessary treatments, in order to coat it with a solid film of a proper biocompatible material, suitable for facilitating the healing of the region which underwent the surgery.

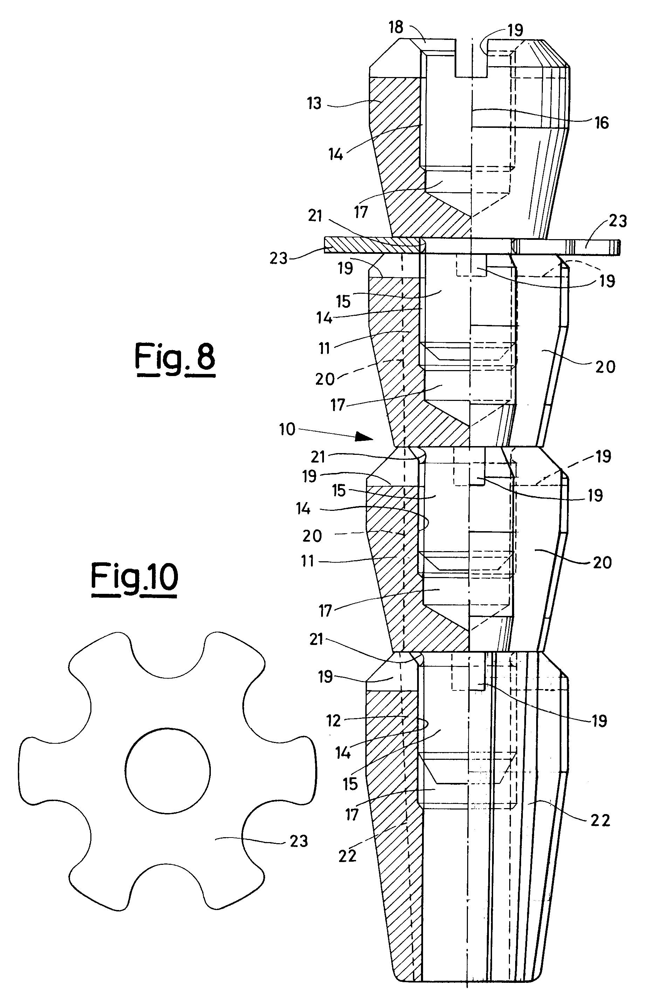

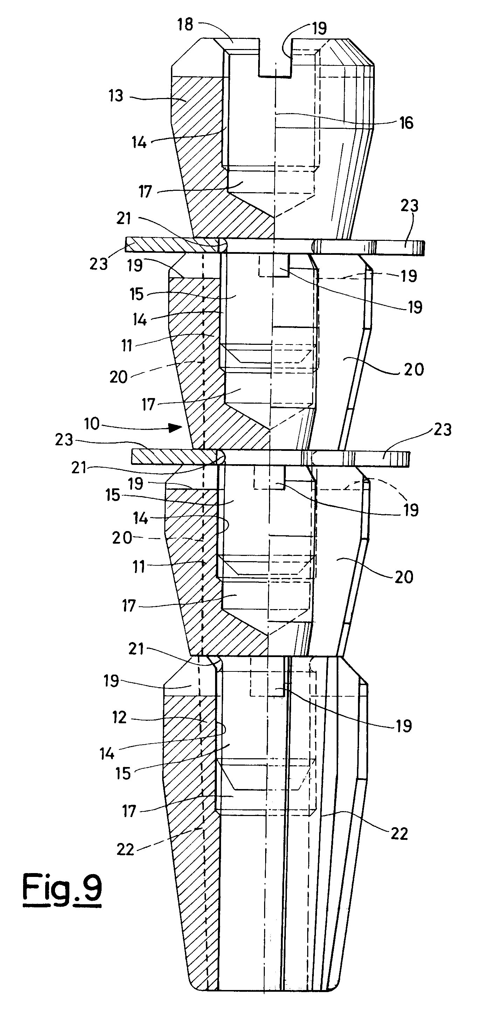

Figures 8 and 9 display the set of modular elements according to the present finding in which between two adjacent elements, one or more stop washer(s) (23) can be interposed, which may have different sizes and shapes, which washers are destined to be housed inside the groove (21).

Said washers (23) perform the purpose of favouring the anchoring of the set of modular elements even in the presence of a too large bone hollow inside which, otherwise, a stable anchoring could not be found unless the same bone hollow is previously reconstituted and/or reconstructed.

On the contrary, the use of said washers (23) allows the instant implantation technique to be adopted.

As one will clearly see from Figures 1, 8 and 9 of the drawings, the set of modular elements realized according to the instant finding takes such a configuration as to define, between adjacent elements, suitable undercuts which facilitate a stable implantation of the whole system inside the interior of the hole provided inside the bone hollow, thus securing an elasticity which is similar to the elasticity of a natural tooth, and cannot be achieved with screws or similar elements of known type, made of one single piece.

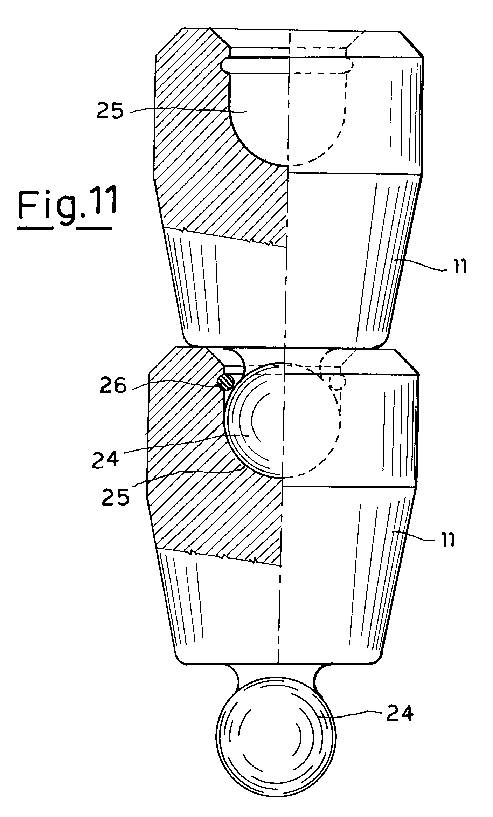

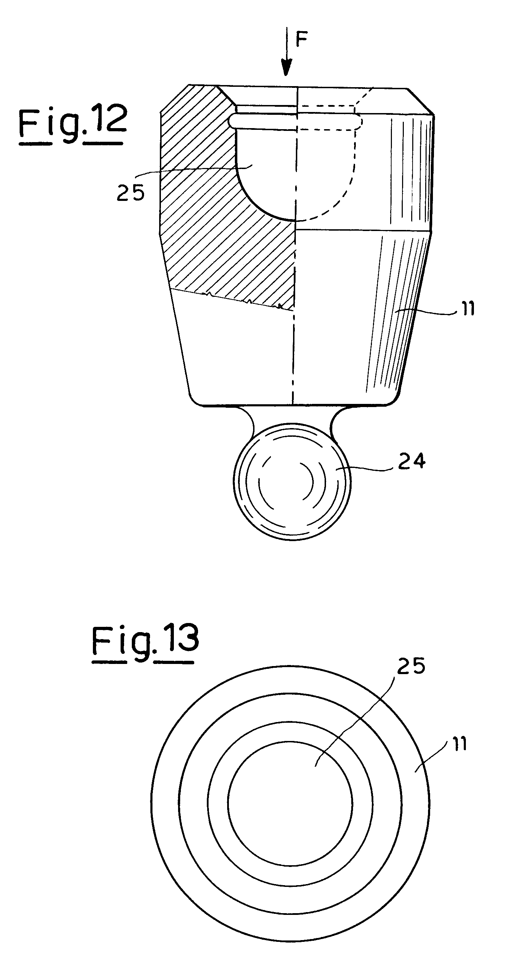

The embodiment of the finding disclosed and illustrated for merely exemplifying, non-limitative purposes, provides for the connection between the modular elements to be realized by means of a screw system; however, of course, other systems for stable intercoupling can be envisaged, however without departing from the purview of protection of the same finding, as defined by the appended claims. Another, also non-limitative, system for connecting the modular elements is illustrated in Figures 11-13.

According to such a system, the modular elements are stably interconnected with one another by means of a quick, male/female spring-like joint.

Said joint can consist, e.g., of a ball head (24) suitable for spring-like entering a complementary hollow (25) inside which said ball head (24) is stably kept by a stop ring (26)

The purpose recited in the preamble to the disclosure is thus achieved.

## Claims

1. Set of modular elements for bone surgery characterized in that it comprises, in combination: a plurality of intermediate elements (11) arranged between a nose element (12) and a head element (13), with each of said intermediate (11) and head (13) elements comprising means for stable mutual intercoupling (14), (15), with the coupling means (15) of each modular element being suitable for being coupled with the coupling means (14) of another, adjacent modular element, said elements (11), (12), (13) having a tapered configuration with side hollows (20), (22) and drive means (19).

2. Set according to claim 1, characterized in that said means for stable mutual intercoupling are constituted by a screw-threaded female portion (14) and a screw-threaded male portion (15).

3. Set according to claim 1, characterized in that said nose element (12) is provided with longitudinal hollows (22) generally having a propeller shape, so as to favour the penetration of the same element into the interior of the bone hollow.

4. Set according to claim 1, characterized in that the side skirt of the head element (13) is not provided with any hollows, so as to favour the spreading of a solid film of a suitable biocompatible material suitable for facilitating the healing of the bone tissue region which underwent the surgery.

5. Set according to claim 1, characterized in that between two adjacent elements, one or more stop washers (23) can be interposed.

6. Set according to claim 1, characterized in that said means for mutual stable intercoupling are constituted by a quick male/female spring like joint.

7. Set according to claim 6, characterized in that said quick joint comprises a ball head (24) suitable for spring-like snapping into a complementary hollow (25) inside which said ball head (24) is stably retained by a stop ring (26).
